# EUROPEAN PATENT APPLICATION

(11) **EP 4 830 639 A1**
(43) Date of publication of application: **28.10.2026**
(21) Application number: 26173542.7
(22) Date of filing: 21.04.2026
(51) Int. Cl.: A61B 8/00, A61B 8/08

(54) **ULTRASOUND DIAGNOSTIC APPARATUS**

(30) Priority: 22.04.2025 JP 2025070497
(71) Applicant: FUJIFILM Corporation, Tokyo 106-8620 (JP)
(72) Inventor: YOSHIKAWA, Hideki, Tokyo (JP); TSUBOTA, Yushi, Tokyo (JP); SONOYAMA, Teruyuki, Tokyo (JP)
(74) Representative: Parker, Andrew James

(57) **Abstract**

An ultrasound diagnostic apparatus detects a homogeneous region 214 of a tissue of a liver from a B-mode image 210, and measures a superficial thickness between a body surface and the homogeneous region 214. Next, the ultrasound diagnostic apparatus determines an upper limit depth of a ROI, that is, a measurement target region based on the superficial thickness. The upper limit depth is obtained using a coefficient determined such that multiple reflection between the body surface and a liver surface is sufficiently attenuated. The ultrasound diagnostic apparatus sets the ROI for various measurements in a range that is within the homogeneous region and that is equal to or deeper than the obtained upper limit depth.

## Description

### BACKGROUND OF THE INVENTION

### 1. Field of the Invention

The present disclosure relates to an ultrasound diagnostic apparatus, and particularly to setting processing of a measurement region of tissue physical properties.

### 2. Description of the Related Art

As measurement modes for diffuse liver disease provided in an ultrasound diagnostic apparatus, shear wave elastography (SWE) and attenuation imaging (ATT) are well known. SWE is a measurement technology of tissue elasticity. In SWE, a shear wave is excited in a tissue in a subject by transmission of a burst wave, and a propagation speed of the shear wave is measured. Then, an elastic modulus (in kPa) of each part in the tissue is calculated from a measurement result of the propagation speed. ATT is a measurement technology of tissue attenuation. In ATT, an amplitude of a transmission signal of a longitudinal wave propagating in a depth direction is measured in a predetermined depth range, and an attenuation rate (in dB/MHz/cm) is calculated based on a result of the measurement. JP2024-117427A discloses a high-performance ultrasound diagnostic apparatus equipped with SWE and ATT.

In order to appropriately perform the measurement in these measurement functions, it is necessary to set a measurement region of interest (ROI) while avoiding structures such as a subcutaneous tissue and a blood vessel. This ROI setting work is manually performed by an examiner in the related art, and requires a lot of time and effort.

JP2024-522311A discloses a method of supporting selection of an ROI suitable for hepatic fat quantification measurement in ultrasound diagnosis of a liver. In this method, for example, a map representing homogeneity of an ultrasound image is generated, and in a case where a user selects an ROI including a heterogeneous fat distribution for hepatic fat quantification measurement, a visual or auditory warning is provided (for example, paragraphs 0010 to 0011 and 0031 to 0032 of JP2024-522311A).

In paragraph 0075 and FIG. 7 of JP2020-138017A, it is shown that an imaging ROI is set in a B-mode image and an elasticity image is superimposed and displayed on the imaging ROI in a screen display of an ultrasound diagnostic apparatus. A first measurement ROI is superimposed and displayed in the elasticity image. In addition, numerical values such as an average value of a shear wave speed and a standard deviation (SD value) in the first measurement ROI are displayed on the screen.

JP2011-143062A discloses an ultrasound diagnostic apparatus that sets a region of interest including a liver boundary line in an ultrasound image related to a liver and calculates an indicator representing a degree of irregularity of the liver boundary line based on an image in the region of interest (for example, paragraphs 0043 to 0048 of JP2011-143062A).

As a method of automatically setting a measurement target region of organ tissue properties, it is conceivable to find a homogeneous region in an organ from an ultrasound image and automatically set a target region in the homogeneous region. However, in a case of simply setting the target region in the homogeneous region, the target region may be adversely affected by multiple reflections of ultrasound waves generated between a body surface and an organ surface in measurement.

### SUMMARY OF THE INVENTION

According to an aspect of the present disclosure, there is provided an ultrasound diagnostic apparatus comprising: a processor, in which the processor is configured to: extract a homogeneous region of an organ from an ultrasound image; obtain a depth of a surface of the organ with respect to a body surface based on the ultrasound image; and set a target region for measurement of tissue physical properties in the homogeneous region based on the depth.

In one aspect, in the setting of the target region, the processor is configured to: obtain an affected depth of multiple reflection according to the depth of the surface of the organ; and set the target region at a position deeper than the affected depth of multiple reflection.

Here, the affected depth of multiple reflection may be obtained based on the depth of the surface and a coefficient of 1 or more, which decreases as the depth of the surface increases.

Further, the coefficient may be obtained according to an exponential function having a base of more than 0 and less than 1.

In addition, the coefficient may be a value of 1.5 to 2.5.

In addition, in one aspect, the measurement includes shear wave elastography and attenuation imaging, and in the setting of the target region, the processor is configured to dispose the target region for the shear wave elastography and the target region for the attenuation imaging to overlap each other.

Here, the target region for the attenuation imaging may be a vertically elongated region.

In addition, in the setting of the target region, the processor may be configured to: determine a position of the target region for the attenuation imaging to be a position deeper than the affected depth of multiple reflection; and set a position of the target region for the shear wave elastography based on the determined position of the target region for the attenuation imaging.

In addition, the measurement may further include backscatter analysis, and in the setting of the target region, the processor may be configured to set a region set as the target region for the attenuation imaging as the target region for the backscatter analysis.

In addition, in another aspect, each time the ultrasound image is obtained by repeatedly executing ultrasound measurement, the processor may be configured to: execute, for the obtained ultrasound image, extraction of the homogeneous region, calculation of the depth of the surface with respect to the body surface, and setting of the target region; execute measurement of the tissue physical properties for the set target region; store data indicating a result of the executed measurement in a storage device; and execute processing of selecting and displaying a predetermined number of pieces of the data satisfying a preset accuracy indicator among the pieces of the data obtained in each measurement stored in the storage device.

In addition, in another aspect, the measurement of the tissue physical properties is at least one of shear wave elastography, attenuation imaging, or backscatter analysis.

In addition, a program according to the present disclosure is a program for causing a computer to execute a process comprising: extracting a homogeneous region of an organ from an ultrasound image; obtaining a depth of a surface of the organ with respect to a body surface based on the ultrasound image; and setting a target region for measurement of tissue physical properties in the homogeneous region based on the depth.

According to the present invention, it is expected that the adverse effect of multiple reflections of ultrasound waves between the body surface and the organ surface in the measurement is reduced as compared with a case where the measurement target region is set only under the condition of being within the homogeneous region of the ultrasound image.

### BRIEF DESCRIPTION OF THE DRAWINGS

FIG. 1 is a diagram illustrating a functional configuration of an ultrasound diagnostic apparatus according to an embodiment.
FIG. 2 is a diagram for describing a relationship between a B-mode image and a region of interest (ROI) for each measurement.
FIG. 3 is a diagram illustrating a relationship between an upper limit depth coefficient of the ROI and a superficial thickness.
FIG. 4 is a diagram illustrating a procedure of measurement processing.
FIG. 5 is a diagram illustrating a procedure of ROI setting.
FIG. 6 is a diagram illustrating a modification example of the procedure of the ROI setting.
FIG. 7 is a diagram illustrating a modification example of the procedure of the measurement processing.

### DESCRIPTION OF THE PREFERRED EMBODIMENTS

Hereinafter, an embodiment (hereinafter, referred to as an embodiment) for carrying out the present disclosure will be described with reference to the drawings.

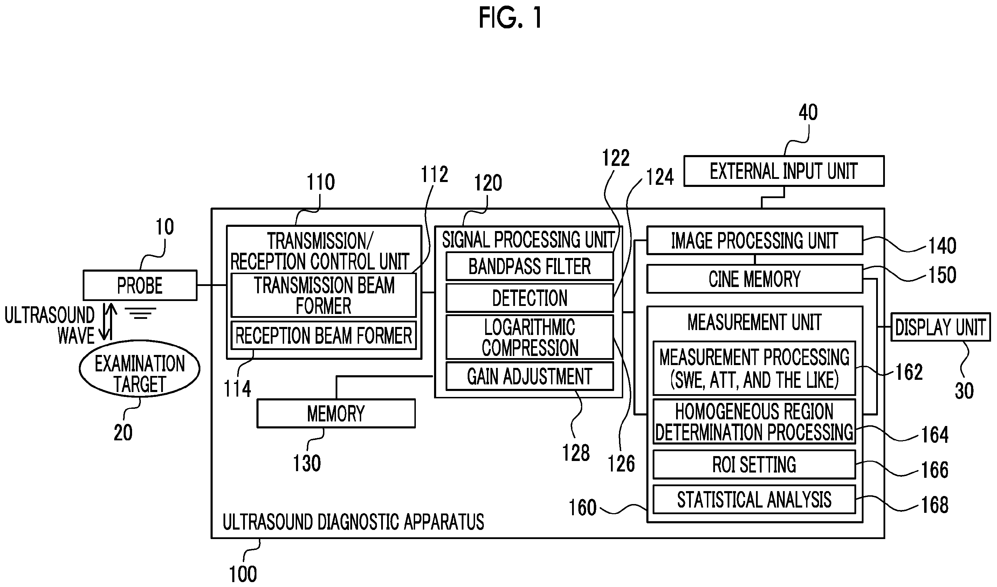

FIG. 1 illustrates a functional configuration of an ultrasound diagnostic apparatus according to the present embodiment. The ultrasound diagnostic apparatus comprises a probe 10, a transmission/reception control unit 110, a signal processing unit 120, a memory 130, an image processing unit 140, a cine memory 150, and a measurement unit 160.

The probe 10 incorporates a plurality of transducers arranged along a transducer surface. The ultrasound is transmitted from each transducer in the probe 10 in accordance with the control of a transmission beam former 112 in the transmission/reception control unit 110, so that a transmission ultrasound beam is formed in an examination target 20. In addition, each transducer in the probe 10 converts a reflected wave from the examination target 20 with respect to the transmission ultrasound beam into an electrical reception signal. A reception beam former 114 in the transmission/reception control unit 110 forms a reception ultrasound beam by performing delay-and-sum on the reception signals output by the respective transducers. The reception beam former 114 outputs a reception beam signal indicating an echo corresponding to the beam for each of the formed reception ultrasound beams. The signal processing unit 120 performs signal processing such as a bandpass filter 122, detection 124, logarithmic compression 126, and gain adjustment 128 on the input reception beam signal. The memory 130 is a storage device used as a work area for processing of the signal processing unit 120. The signal output by the signal processing unit 120 is input to the image processing unit 140 and the measurement unit 160.

The image processing unit 140 performs known image processing using an image filter or the like on the signal output by the signal processing unit 120 to generate an ultrasound image such as a B-mode image. The generated ultrasound image is stored in a cine memory 150 and is displayed on the display unit 30.

The measurement unit 160 executes processing for measuring tissue physical properties of an organ, such as SWE or ATT. The processing executed by the measurement unit 160 includes measurement processing 162, homogeneous region determination processing 164, ROI setting 166, statistical analysis 168, and the like.

The measurement processing 162 executes control and data analysis for measurement of ATT, SWE, or the like. For example, the measurement processing 162 controls transmission and reception of an ultrasound beam for measurement. In addition, the measurement processing 162 executes known data analysis for ATT or SWE on the reception signal obtained by the transmission and reception.

The homogeneous region determination processing 164 determines or detects a homogeneous region from the ultrasound image such as a B-mode image. The homogeneous region is a region in which a property of the image is considered to be homogeneous. A region in which a speckle pattern is observed (hereinafter, referred to as a speckle region) is a representative example of the homogeneous region. The speckle pattern is a granular pattern formed by interference between scattering signals of ultrasound waves caused by microscopic scatterers scattered in a biological tissue. For example, in a case of the liver, a frequency band and intensity of scattering change due to a difference in tissue state, for example, a difference between a normal liver and a liver with progressed fibrosis, and the speckle pattern also changes. The homogeneous region is not limited to the speckle region. In a case in which a pattern is periodically repeated in a certain period with a certain degree of fineness over a range of a certain degree of width (for example, several square centimeters), the range corresponds to the homogeneous region.

Various methods have been proposed in the related art for determining the homogeneous region, and various methods will be developed in the future. The homogeneous region determination processing 164 may use any of such methods in the related art or methods to be developed in the future. For example, the homogeneous region determination processing 164 may determine the homogeneous region using a local feature amount of the image, or may use an artificial intelligence technology such as a machine learning model.

The ROI setting 166 sets an ROI for tissue physical property measurement in a region of the organ to be measured in the ultrasound image. Here, the ROI is set in the determined homogeneous region. In the present embodiment, the ROI is set based on a superficial thickness between the probe 10 and the organ to be measured. The superficial thickness is a thickness of a body surface tissue between a body surface in contact with the probe 10 and the organ to be measured. Therefore, it can be said that the superficial thickness is a depth of a surface of the organ with respect to the body surface. The ROI is set based on the superficial thickness in order to sufficiently reduce an influence of multiple reflections of the ultrasound waves between the body surface and the organ surface.

In general, the ultrasound waves transmitted from the body surface propagate while being repeatedly scattered or reflected by the microscopic scatterers in the living body. However, at a boundary between the probe and the body surface tissue (for example, the fascia or the collagen membrane) or the liver surface, multiple reflection in which the ultrasound signal reciprocates between the boundaries occurs. The multiple reflection causes a virtual image in the ultrasound measurement. Here, it is known in principle that the virtual image occurs at a depth that is an integral multiple of a depth of the boundary at which the multiple reflection occurs. In addition, the ultrasound signal is attenuated according to an effective propagation distance in a process of repeatedly performing the multiple reflection. As the superficial thickness is larger, the effective propagation distance due to the multiple reflection is longer, and accordingly, the influence of the virtual image due to the multiple reflection is also smaller.

Therefore, the ROI setting 166 obtains the superficial thickness from the ultrasound image, determines a depth range suitable for the ROI based on a predetermined rule from the superficial thickness, and sets the ROI within the depth range. Details of the ROI setting will be described below.

The statistical analysis 168 executes the statistical analysis on the measurement results of the ATT, the SWE, and the like performed by the measurement processing 162. The analysis performed by the statistical analysis 168 will be described below.

The display unit 30 displays a display screen generated by the ultrasound diagnostic apparatus 100.

The external input unit 40 receives an input from the user for the ultrasound diagnostic apparatus 100.

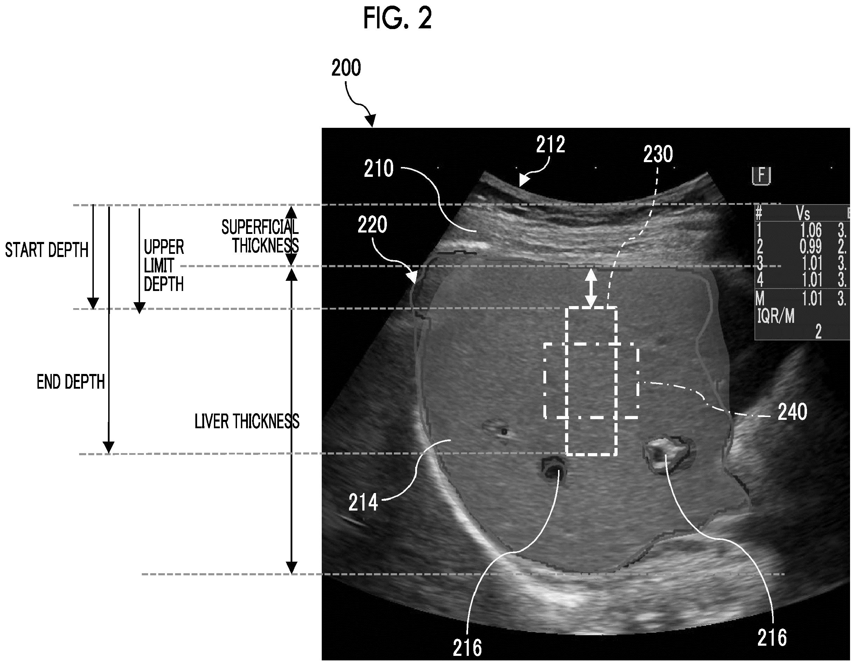

Next, the processing of the ROI setting 166 will be further described with reference to FIG. 2.

FIG. 2 shows a display window 200 in which a B-mode image 210 of the liver is displayed. The B-mode image 210 is obtained by the convex probe 10. An upper end side 212 of the B-mode image 210 has an arc shape according to a shape of the transducer surface of the probe 10, and represents a body surface in contact with the transducer surface of the probe 10. A direction from the top to the bottom in the figure is a symmetry axis of an annular fan-shaped B-mode image 210, in other words, a direction of a normal line at a position of a center of a diameter of the transducer surface of the probe 10. Hereinafter, the normal line will be referred to as a diameter center line. A homogeneous region 214 of the liver tissue is detected by the homogeneous region determination processing 164. In addition, a contour 220 of the liver including the homogeneous region 214 may be detected by another analysis technique. A structure 216 such as a blood vessel in the liver is not included in the detected homogeneous region 214.

On the diameter center line of the B-mode image 210, a distance from the body surface to a liver surface close to the body surface is a superficial thickness. Here, the contour of the homogeneous region 214 may be the liver surface. Alternatively, the contour 220 may be the liver surface. In addition, a width between an upper end and a lower end of the liver surface on the diameter center line is a liver thickness.

By the ROI setting 166, the ROI 230 for ATT and the ROI 240 for SWE are set in the homogeneous region 214 of the liver.

Since the attenuation of the ultrasound wave along the depth direction is measured in the ATT, the ROI 230 for ATT needs to have a width of about 10 to 40 mm along the depth direction. In the following example, as an example, it is assumed that the width of the ROI 230 for ATT in the depth direction is 30 mm. Since the measurement of the ATT can be performed with only one scan line in principle, there is no particular principle requirement for the width of the ROI 230 for ATT in the lateral direction (in other words, the scanning direction). However, in a case in which the measurement is executed with a plurality of scan lines in the lateral direction in order to compensate for the accuracy of the attenuation measurement, the width of the ROI 230 for ATT in the lateral direction is often set to about 10 to 20 mm. In addition, in a case in which the two-dimensional distribution of the attenuation of the ultrasound wave is observed, the width in the lateral direction may be set to be wider. In the following, as an example, it is assumed that the width of the ROI 230 for ATT in the lateral direction is 10 mm. It should be noted that this is merely an example, and the size of the ROI 230 for ATT is appropriately determined according to various conditions of the ATT measurement.

The ROI 240 for SWE needs to be limited to a range in which the excited shear wave can be detected. Therefore, the range in which the shear wave can be detected changes depending on the excitation method, the power of the ultrasound wave used for excitation, and the like, but under normal conditions, the range is about ±10 mm in the depth direction and about ±10 mm in the lateral direction with the excitation location as a starting point. Therefore, under normal conditions, the width of the ROI 240 for SWE in both the depth direction and the lateral direction is set to about 20 mm or less. In the following, as an example, the width of the ROI 240 for SWE in both the depth direction and the lateral direction is set to 20 mm. It should be noted that this is merely an example, and the size of the ROI 240 for SWE is appropriately determined according to various conditions of the SWE measurement.

The conditions of the ROI for each measurement shown above are for the case of assuming the simplest measurement. In a case of assuming more advanced measurement, the size and shape of the ROI may change. For example, the ROI may be expanded to extend the measurement to a wider range.

The ROI 230 for ATT and the ROI 240 for SWE are set at positions deeper than an upper limit depth calculated based on a superficial thickness as viewed from a body surface (represented by an upper end side 212 of the B-mode image 210 in the drawing). The upper limit depth is a depth representing an upper limit of an upper end position of the ROI for tissue measurement. A range shallower than the upper limit depth is a range in which the influence of multiple reflections of the ultrasound waves between the body surface and the liver surface is strong. Therefore, the upper limit depth can be said to be a depth at which the multiple reflection has an influence, in a sense that the influence of the multiple reflection is so strong that it cannot be allowed.

The start depth and the end depth shown in the drawing are depths of an upper end and a lower end of the ROI 230 for ATT. Therefore, the ROI 230 for ATT is set such that the start depth is equal to or deeper than the upper limit depth described above. By setting the ROI such as the ROI 230 for ATT in a range deeper than the upper limit depth, the influence of the multiple reflection on the measurement can be kept within an allowable range. In addition, although merely an example, the illustrated example is an example in a case where a rule of setting the start depth of the ROI 240 for SWE to a position deeper than the start depth of the ROI 230 for ATT is followed.

In the example shown in FIG. 2, both the ROI 230 for ATT and the ROI 240 for SWE are set not to include, in the inside thereof, a portion other than the homogeneous region 214 (for example, a structure 216).

The example shown in FIG. 2 is an example in a case where the start depth of the ROI 230 for ATT matches the upper limit depth obtained from the superficial thickness.

As described above, the upper limit depth of the ROI is obtained based on the superficial thickness. For example, the upper limit depth is obtained according to the following Equation (1).$Upper limit depth = superficial thickness \times upper limit depth coefficient$ (1)

The upper limit depth coefficient is a positive value. In consideration of the above-described property that the influence of the virtual image due to the multiple reflection is smaller as the superficial thickness is larger, it is considered that the upper limit depth coefficient is expressed by a function that is smaller as the superficial thickness is larger. Here, since the attenuation of the strength of the virtual image due to the magnitude of the superficial thickness is the attenuation due to the increase in the propagation distance of the ultrasound wave, it can be said that the function representing the upper limit depth coefficient is preferably a function similar to an exponential function in which the numerical value x of the base is 0 < x < 1, rather than a function that linearly decreases with respect to the increase in the superficial thickness. In addition, since the attenuation of the ultrasound intensity with respect to the increase in the propagation distance is larger as the frequency of the transmitted ultrasound wave is higher, it is considered that the upper limit depth coefficient is a function that is smaller as the frequency is higher. In consideration of the above, it is considered that, for example, a function represented by the following Equation (2) is used as the upper limit depth coefficient y.$y = Aexp \left(-azf\right)$ (2)

In Equation (2), A and a are constant coefficients, z is the superficial thickness, and f is the frequency.

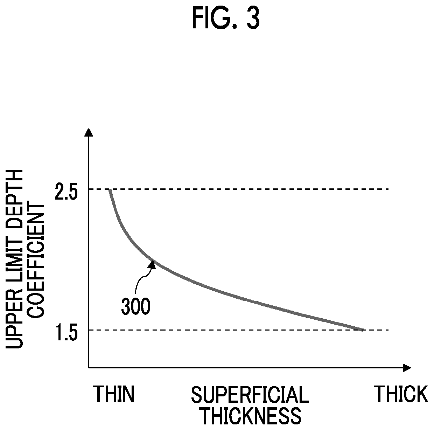

FIG. 3 schematically shows a function 300 of Equation (2) in a case in which the frequency f is fixed. Although merely an example, the function of FIG. 3 takes values in a range from 1.5 to 2.5 in a range of the superficial thickness generally considered. Since the detection signal of the ultrasound wave until the ultrasound wave reciprocates twice between the body surface and the liver surface is the virtual image signal in which the influence of the multiple reflection is most strongly exhibited, basically, it can be said that a range of a depth obtained by doubling the superficial thickness is a depth in which the influence of the multiple reflection is most strongly exhibited. However, as described above, since the propagation distance of the ultrasound wave is longer as the superficial thickness is larger and the virtual image of the multiple reflection is weaker, the upper limit coefficient is larger than 2 in consideration of the multiple reflection exceeding two reciprocations in a range in which the superficial thickness is thin, and the upper limit coefficient is smaller than 2 in a range in which the superficial thickness is thick.

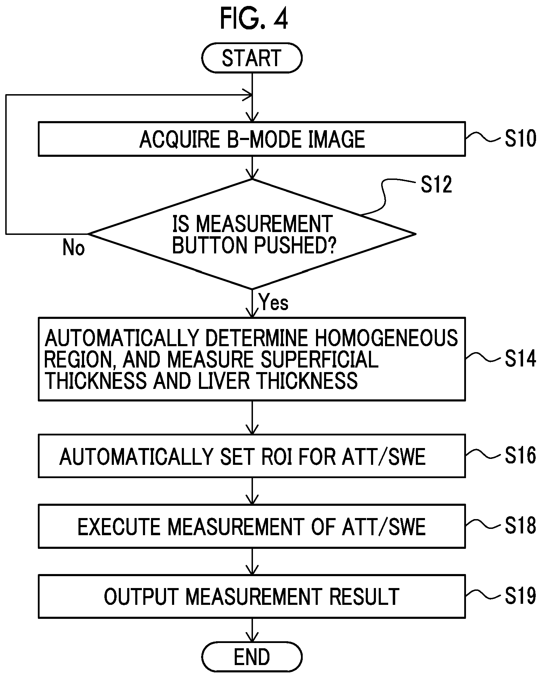

Next, an example of a processing procedure of the measurement unit 160 will be described with reference to FIG. 4.

The examiner brings the probe 10 into contact with the subject to image a range including an organ (for example, the liver) to be examined. By this operation, a B-mode image in which the target organ is included in the visual field is acquired (S10). The measurement unit 160 waits for an instruction to start the measurement to be input (S12). The examiner searches for a desired cross section by changing a position or a direction in which the probe 10 is brought into contact with the subject while viewing the B-mode image displayed on the display unit 30. At a point in time at which the desired cross section is displayed on the display unit 30, the examiner inputs the instruction to start the measurement from the external input unit 40. As a result, the determination result in step S12 is Yes. In response to this, the B-mode image displayed on the display unit 30 is in a temporarily stopped state, and the measurement unit 160 starts the measurement processing for the image.

In the measurement processing, first, homogeneous region determination processing 164 automatically determines a homogeneous region in the target B-mode image and measures the superficial thickness and the liver thickness (S14).

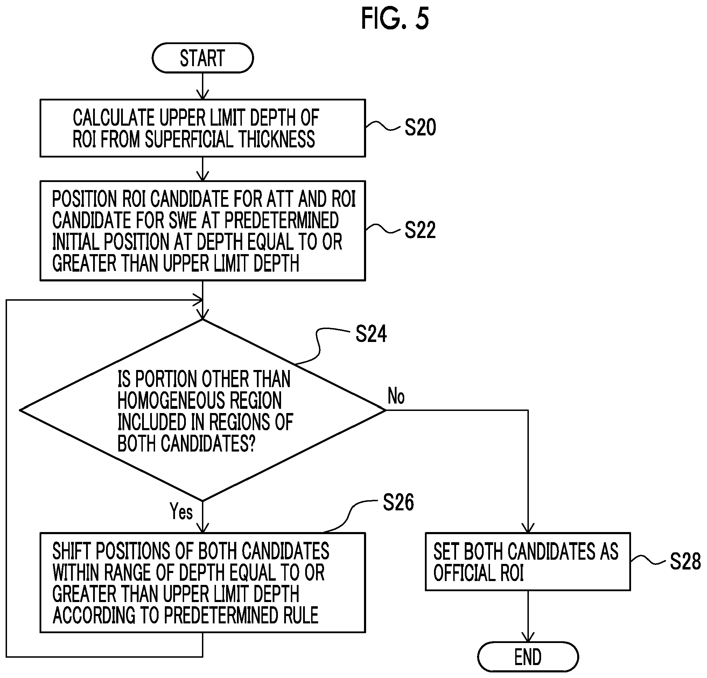

Next, ROI setting 166 sets the ROI 230 for ATT and the ROI 240 for SWE based on the homogeneous region, the superficial thickness, the liver thickness, and the like (S16). In step S16, an example of detailed processing will be described later with reference to FIG. 5 and the like.

Next, the measurement processing 162 executes the measurement of ATT for the ROI 230 for ATT and executes the measurement of SWE for the ROI 240 for SWE (S18). In each measurement, the transmission and reception of the ultrasonic wave for the measurement are performed, and the measurement is performed based on the reception signal. Then, the measurement processing 162 outputs the measurement results obtained by the measurements (S19). The output may be, for example, display on the display unit 30 or data registration in a patient database.

Next, an example of a detailed procedure of step S16 will be described with reference to FIG. 5. In this procedure, the ROI setting 166 calculates the upper limit depth of the ROI by a method such as using a function as in Expression (2) described above from the superficial thickness obtained in step S14 (S20). Next, the ROI setting 166 positions the candidate for the ROI 230 for ATT and the candidate for the ROI 240 for SWE at a predetermined initial position at a depth equal to or greater than the upper limit depth (S22).

The initial position in this case is, for example, a position at the upper limit depth from the body surface on the diameter center line of the probe 10. The upper end side center of the ROI 230 for ATT, which has a larger width in the depth direction among the two ROIs, is positioned at the initial position. In this example, the relative positional relationship between the ROI 230 for ATT and the ROI 240 for SWE is fixed. For example, it is assumed that the ROI 240 for SWE is determined such that the center position in the lateral direction coincides with the center position of the ROI 240 for ATT and the start depth is a position deeper than the start depth of the ROI 230 for ATT by a predetermined length. Therefore, in this example, in a case where the ROI 230 for ATT is positioned, the position of the ROI 240 for SWE is also automatically determined.

Next, the ROI setting 166 determines whether or not a portion other than the homogeneous region (for example, the structure 216) is included in the regions of both the candidates for the ROI 230 for ATT and the ROI 240 for SWE positioned in step S22 (S24).

Here, since the measurement of the tissue physical properties such as ATT and SWE targets the homogeneous region, it is difficult to perform accurate measurement in a case where a portion other than the homogeneous region is included in the ROI. Therefore, in a case where the result of the determination in step S24 is Yes, the ROI setting 166 shifts the positions of both the candidates for the ROI 230 for ATT and the ROI 240 for SWE within a range of a depth equal to or greater than the upper limit depth according to a predetermined rule (S26). Then, the ROI setting 166 repeats the determination in step S24 for both the candidates after the shifting. The loops of steps S24 and S26 are repeated until the determination in step S24 is No, that is, until only the homogeneous region is included in both the candidates.

In a case where the result of the determination in step S24 is No, the ROI setting 166 sets the positions of both the candidates at that point in time as the positions of the official ROI 230 for ATT and the official ROI 240 for SWE (S28).

In the search in step S26 according to the predetermined rule, for example, first, the determination in step S24 is repeated while variously changing the lateral positions of both the candidates while maintaining the depth direction position of the upper end side of the candidate for the ROI 230 for ATT at the upper limit depth described above. In a case where the result of the determination in step S24 is not No even in a case where the lateral position is changed in any way, both the candidates are moved downward by a predetermined amount in the depth direction, and a position where the result of the determination in step S24 is No is searched for while both the candidates are variously moved laterally at the depth direction position. In a case where the result of the determination in step S24 is not No even in a case where the lateral position is changed in any way at that depth, both the candidates are further moved downward by a predetermined amount in the depth direction, and the same search is repeated.

The appropriate ROI 230 for ATT and the appropriate ROI 240 for SWE are set by the procedure in FIG. 5.

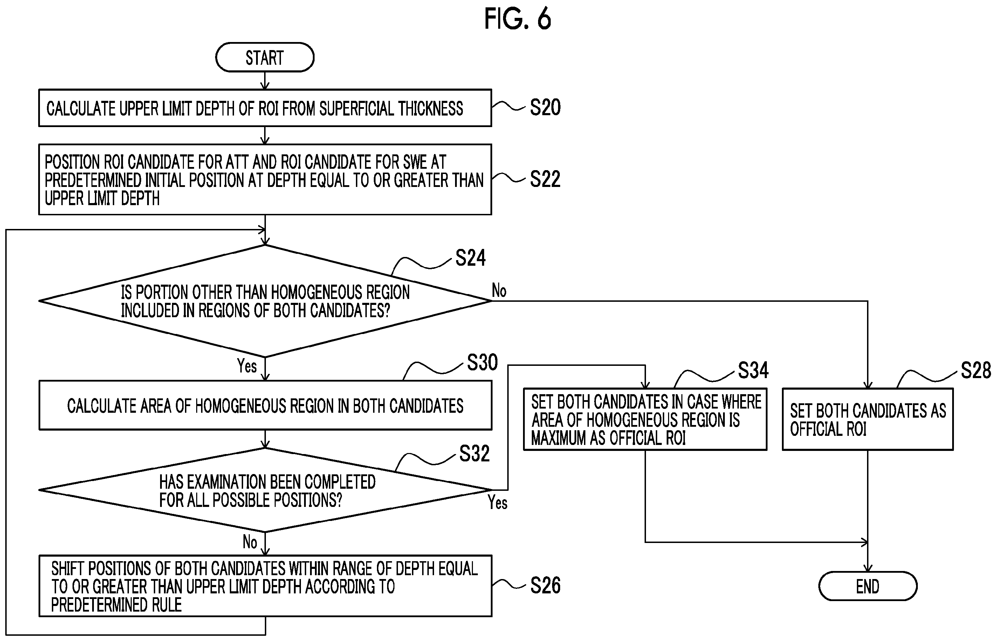

Next, a modification example of the detailed procedure of step S16 will be described with reference to FIG. 6. In the procedure in FIG. 5, there may be a case where the ROI cannot be made to have only a homogeneous region even in a case where the positions of the ROI 230 for ATT and the ROI 240 for SWE are changed in any way. In the procedure in FIG. 6, even in such a case, the ROI 230 for ATT and the ROI 240 for SWE can be set at a position where the homogeneous region is as wide as possible. In FIG. 6, the same steps as the steps in the procedure in FIG. 5 are denoted by the same reference numerals, and the overlapping description will be omitted.

In the procedure in FIG. 6, in a case where the result of the determination in step S24 is Yes, the ROI setting 166 obtains the area of the homogeneous region in both the candidates (or the ratio of the area of the homogeneous region to the total area of both the candidates) and stores the area in association with the position information of both the candidates (S30). Next, the ROI setting 166 determines whether or not the examination has been completed for all the positions that can be taken by both the candidates in a range deeper than the upper limit depth (S32). In a case where the result of the determination is No, the ROI setting 166 shifts the positions of both the candidates according to a predetermined rule within a range of a depth equal to or deeper than the upper limit depth (S26), and repeats the determination in step S24 again.

In a case where the result of the determination in step S32 is Yes, the ROI setting 166 sets the ROI 230 for ATT and the ROI 240 for SWE at a position indicated by the position information of the combination having the maximum area among the combinations of the position information and the area stored in step S30 in the repetition so far (S34).

According to the procedure in FIG. 6, both the ROIs can be set at a position where the area of the homogeneous region is the maximum.

The processing procedure in FIG. 6 assumes a work procedure in which the examiner inputs an instruction to start the measurement after confirming the cross section to be measured while viewing the ultrasound image displayed as a video. The ROI is automatically set in response to the instruction to start the measurement, and the measurement is executed for the ROI. However, such a work procedure is merely an example.

As another example, the ROI setting 166 may automatically set and display the ROI for the tissue physical property measurement on the ultrasound image displayed as a video. For example, in a case where the user gives an instruction to execute the automatic ROI display, the ROI setting 166 automatically sets the ROI for the frame of the video. In this case, the ROI displayed to be superimposed on the ultrasound image can change in accordance with the change in the ultrasound image as the video. The examiner gives an instruction to start the measurement at a timing suitable for the measurement while viewing the video of the ultrasound image on which the ROI is superimposed. In response to the instruction, the measurement unit 160 executes the measurement for the ROI displayed at the timing at which the instruction to start the measurement is received. It should be noted that, as the timing suitable for the measurement, for example, a timing at which the ultrasound image to be displayed is in a substantially stationary state as a result of the subject stopping breathing in response to a signal to hold the breath is considered.

In this example, the ROI setting 166 may automatically set the ROI by executing the same processing procedure as the procedure in FIG. 6 for the frame of the video. Such automatic setting of the ROI may be executed for each frame of the video. In addition, it is assumed that the processing capacity of the processor is insufficient to automatically set the ROI for all the frames, and the automatic setting of the ROI may be executed, for example, for each of several frames.

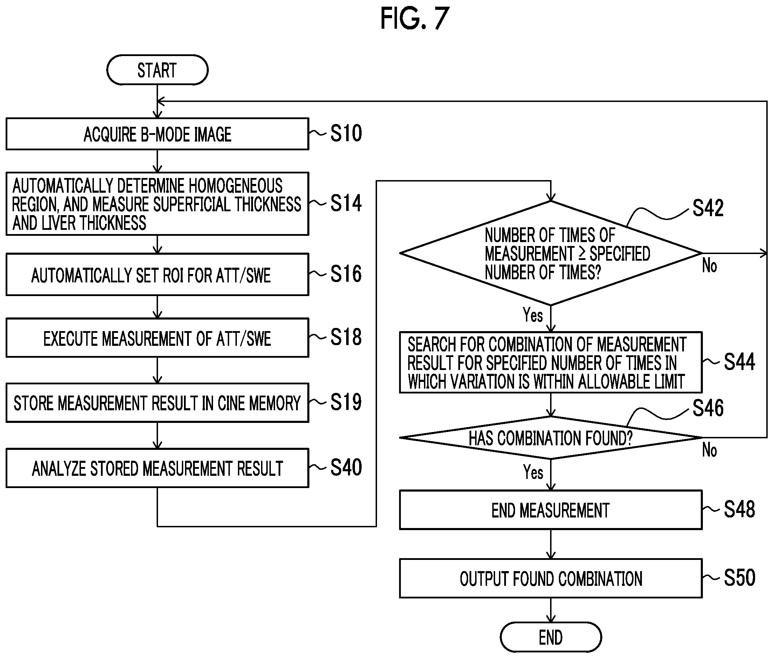

Next, a modification example of the processing procedure of the measurement unit 160 will be described with reference to FIG. 7. In FIG. 7, the same steps as the steps in the procedure in FIG. 4 are denoted by the same reference numerals.

Since there is variation in the measurement results of the ATT and the SWE due to various factors, it is necessary to repeat the measurement a certain number of times to obtain a reliable measurement result and to extract a measurement result with high reliability. In the procedure in FIG. 7, the measurement unit 160 automatically executes such repeated measurements.

The procedure in FIG. 7 is started in response to a trigger in which the examiner inputs an instruction to start the measurement to the ultrasound diagnostic apparatus 100. In this procedure, in a case in which the B-mode image is acquired by the transmission and reception of the ultrasound waves from the probe 10 (S10), the measurement unit 160 obtains the homogeneous region, the superficial thickness, and the like from the B-mode image (S14), and sets the ROI 230 for the ATT and the ROI 240 for the SWE (S16). Next, the measurement unit 160 executes the measurement of the ATT and the SWE using the set ROIs (S18), and stores the measurement result in the cine memory 150 in association with, for example, the B-mode image acquired in S10 (S19).

Next, the statistical analysis 168 in the measurement unit 160 performs numerical analysis on the current measurement result stored in the cine memory 150 (S40). In this analysis, for example, the average of the measurement values in the corresponding ROI is calculated for each of the ATT and the SWE. That is, in the ATT, the attenuation coefficient of the ultrasound wave at each point is measured, but in step S40, the average value of the attenuation coefficients in the ROI 230 for the ATT is obtained. Similarly, the average value of the SWE measurement values (for example, the measurement values of the propagation speed of the shear wave) in the ROI 240 for the SWE is calculated. As a result, the average values of the attenuation coefficient and the propagation speed in the current measurement are obtained.

Next, the statistical analysis 168 determines whether or not the number of times of the measurement performed so far has reached the specified number of times after receiving the instruction to start the measurement (S42). In a case in which the result of this determination is No, the measurement unit 160 returns to step S10, acquires the next B-mode image, and repeats the processing of steps S14 to S42.

In a case in which the determination result in step S42 is Yes, the measurement unit 160 searches for a combination of the measurement results for the specified number of times in which the statistical value indicating the variation is within the predetermined allowable limit, from the stored measurement results (S44).

As the statistical value indicating the variation, for example, IQR/median may be used. Here, an interquartile range (IQR) is a difference (Q3 - Q1) between a third quartile Q3 and a first quartile Q1 of the data in the population. The quartile is data that serves as a delimiter in a case in which the data in the population is arranged in ascending order and the arrangement is divided into four equal parts from the viewpoint of the number of data. Ordinal numbers of first, second, and third are assigned to the quartiles in ascending order. The IQR/median is a value obtained by dividing the IQR by the median (that is, the central value) of the population, and is expressed as a percentage.

That is, in step S44, the statistical analysis 168 selects a combination of the measurement results for the specified number of times from the measurement results stored from the start of the procedure in FIG. 7 to the current point in time (referred to as a "population" below), and calculates the IQR/median for the selected combination. Next, the statistical analysis 168 determines whether or not the calculated IQR/median is within a predetermined allowable limit (for example, "10%"). The allowable limit for the IQR/median is an example of an accuracy indicator for the measurement. In a case in which a result of the determination is No (that is, the IQR/median is larger than the allowable limit), the statistical analysis 168 selects another combination of the measurement results for the specified number of times from the population, and repeats the same determination.

In the repetition, in a case in which a combination in which the IQR/median is equal to or less than the allowable limit is not found from the population, a determination result in step S46 is No. In this case, the measurement unit 160 returns to step S10, acquires a new B-mode image, and executes the processing of steps S14 to S44 on the B-mode image.

On the other hand, in a case in which a combination in which the IQR/median is equal to or less than the allowable limit is found from the population, the determination result in step S46 is Yes. In this case, the measurement unit 160 ends the measurement (S48) and outputs the found combination in a form of screen display, database storage, or the like (S50).

In step S50, for example, the value of the measurement result (for example, the average value of the attenuation coefficient and the average value of the shear wave velocity) and the thumbnail of the B-mode image corresponding to the value may be displayed in association with each other for each of the measurement results included in the found combination. A link for calling the original B-mode image may be set in the thumbnail.

A specific example of the processing according to the procedure of FIG. 7 will be described. In this example, it is assumed that the specified number of times is five. At a stage in which the measurement is executed five times from the measurement start, the first variation value (for example, IQR/median) for the combination of the five measurement values is calculated. It is assumed that the variation value is greater than the allowable limit. In this case, the sixth measurement is executed. In a case in which the sixth measurement value is input, a new combination of five measurement values is created by excluding the measurement value that is statistically most outlying from the past five measurement values used for calculating the previous variation value and the current measurement value, that is, the sixth measurement value. Then, the variation value for the new combination is calculated, and the combination and the variation value are stored in the memory. By repeating this step, the combination of the measurement values for the specified number of times is updated in a direction in which the variation value is decreased. Finally, the measurement is automatically ended at a stage in which a condition specified in advance, that is, the variation value is within a predetermined allowable limit, and the result is displayed on, for example, the display unit 30.

In the above example, since two types of measurements, that is, ATT and SWE, are performed, the search in step S44 and the determination in step S46 are performed for each of the two types. In a case in which the determination result in step S46 is Yes for one type, the measurement of the type may be ended, and the measurement of the other type may return to step S10 and be repeated.

According to the procedure in FIG. 7, the examiner can automatically complete the measurement by simply applying the probe 10 to the examination target. That is, not only is the work of setting the ROI while identifying the homogeneous region unnecessary, but the examiner himself/herself does not need to select data with a small statistical variation. Therefore, it is possible to expect an accurate and short examination.

It should be noted that, as the measurement of the tissue physical properties using the ultrasound, for example, BSC is known in addition to the ATT and the SWE. BSC is an abbreviation for backscatter coefficient. BSC is a technique for evaluating the unevenness of the tissue, and measures the scattering intensity of the transmission and reception signal of the ultrasound from the tissue. BSC is introduced in, for example, Keith A. Wear et al., "US backscatter for Liver Fat Quantification: An AIUMRSNA QIBA Pulse-Echo Quantitative Ultrasound Initiative", Radiology: Volume 305: Number 3 - December: 526-537 pages: 2022: radiology.rsna.org, or "Development of ultra-high-precision ultrasound evaluation by scatterer structure analysis in a living body" by Kazuki Tamura et al., Reports of Research Results of Tateisi Science and Technology Foundation (No. 28) 2019.

Since BSC analyzes the longitudinal wave of the ultrasound, in principle, one-dimensional signal along a single scan line is sufficient. Therefore, the ROI of the BSC can be shared with the ROI of the ATT targeting the same one-dimensional signal. Therefore, in the above-described embodiment, the BSC may be further measured using the ROI of the ATT.

In addition, the measurement of the ATT, the SWE, the BSC, and the like described above is merely an example. The method of the present embodiment can also be applied to a measurement method of tissue physical properties other than the above.

In the present embodiment, each type of processing is executed by any computer. In addition, any computer may execute these processes using a processor as hardware, a program as software, or a combination thereof. In such a case, the processor is configured to execute various types of processes in the present embodiment in cooperation with the program, and may function as each unit or each means in the present embodiment. Further, the execution order of the processes by the processor is not limited to the order described and may be changed as appropriate. Any computer may be a general purpose computer, a special purpose computer, a workstation, or other system capable of executing each process.

The processor may be configured by one or more kinds of hardware, and the type of hardware is not limited. For example, the processor may be configured using hardware such as a central processing unit (CPU), a micro-processing unit (MPU), a programmable logic device such as a field-programmable gate array (FPGA), a dedicated circuit for executing specific processing, such as an application-specific integrated circuit (ASIC), a graphics processing unit (GPU), or a neural processing unit (NPU). Additionally, the type of hardware may be a combination of different types of hardware. In a case where a plurality of pieces of hardware are configured to execute one or a plurality of types of processing of a processor, the plurality of pieces of hardware may be present in apparatuses physically separated from each other, or may be present in the same apparatus. In addition, in any of the embodiments, the order of each processing by the processor is not limited to the above order, and may be appropriately changed. The hardware is configured by an electric circuit (circuitry) in which circuit elements such as semiconductor elements are combined.

Further, the program may be software, such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, storage media or other storages). The program may be divided and stored in a plurality of non-transitory computer-readable media present in apparatuses physically separated from each other. The program code or the code segments may represent any combination of procedures, functions, subprograms, routines, subroutines, modules, software packages, classes, instructions, data structures, or program statements. The program code or code segments may be connected to other code segments or to hardware circuits by transmitting and receiving information, data, arguments, parameters, or contents of memory.

### Explanation of References

10: probe
30: display unit
40: external input unit
100: ultrasound diagnostic apparatus
110: transmission/reception control unit
120: signal processing unit
130: memory
140: image processing unit
150: cine memory
160: measurement unit
162: measurement processing
164: homogeneous region determination processing
166: ROI setting
168: statistical analysis

## Claims

1. An ultrasound diagnostic apparatus comprising:
a processor,
wherein the processor is configured to:
extract a homogeneous region of an organ from an ultrasound image;
obtain a depth of a surface of the organ with respect to a body surface based on the ultrasound image; and
set a target region for measurement of tissue physical properties in the homogeneous region based on the depth.

2. The ultrasound diagnostic apparatus according to claim 1,
wherein in the setting of the target region, the processor is configured to:
obtain an affected depth of multiple reflection according to the depth of the surface of the organ; and
set the target region at a position deeper than the affected depth of multiple reflection.

3. The ultrasound diagnostic apparatus according to claim 2,
wherein the affected depth of multiple reflection is obtained based on the depth of the surface and a coefficient of 1 or more, which decreases as the depth of the surface increases.

4. The ultrasound diagnostic apparatus according to claim 3,
wherein the coefficient is obtained according to an exponential function having a base of more than 0 and less than 1.

5. The ultrasound diagnostic apparatus according to either claim 3 or claim 4,
wherein the coefficient is a value of 1.5 to 2.5.

6. The ultrasound diagnostic apparatus according to any one of claims 1 to 5,
wherein the measurement includes shear wave elastography and attenuation imaging, and
in the setting of the target region, the processor is configured to dispose the target region for the shear wave elastography and the target region for the attenuation imaging to overlap each other.

7. The ultrasound diagnostic apparatus according to any one of claims 1 to 6,
wherein the target region for the attenuation imaging is a vertically elongated region.

8. The ultrasound diagnostic apparatus according to any one of claims 1 to 7,
wherein in the setting of the target region, the processor is configured to:
determine a position of the target region for the attenuation imaging at a position deeper than the affected depth of multiple reflection; and
set a position of the target region for the shear wave elastography based on the determined position of the target region for the attenuation imaging.

9. The ultrasound diagnostic apparatus according to any one of claims 1 to 8,
wherein the measurement further includes backscatter analysis, and
in the setting of the target region, the processor is configured to set a region set as the target region for the attenuation imaging as the target region for the backscatter analysis.

10. The ultrasound diagnostic apparatus according to any one of claims 1 to 9,
wherein each time the ultrasound image is obtained by repeatedly executing ultrasound measurement, the processor is configured to:
execute, for the obtained ultrasound image, extraction of the homogeneous region, calculation of the depth of the surface with respect to the body surface, and setting of the target region;
execute measurement of the tissue physical properties for the set target region;
store data indicating a result of the executed measurement in a storage device; and
execute processing of selecting and displaying a predetermined number of pieces of the data satisfying a preset accuracy indicator among the pieces of the data obtained in each measurement stored in the storage device.

11. The ultrasound diagnostic apparatus according to any one of claims 1 to 10,
wherein the measurement of the tissue physical properties is at least one of shear wave elastography, attenuation imaging, or backscatter analysis.

12. A program for causing a computer to execute a process comprising:
extracting a homogeneous region of an organ from an ultrasound image;
obtaining a depth of a surface of the organ with respect to a body surface based on the ultrasound image; and
setting a target region for measurement of tissue physical properties in the homogeneous region based on the depth.

13. A method for processing an ultrasound image comprising:
extracting a homogeneous region of an organ from an ultrasound image;
obtaining a depth of a surface of the organ with respect to a body surface based on the ultrasound image; and
setting a target region for measurement of tissue physical properties in the homogeneous region based on the depth.
